(19) Europäisches Patentamt
European Patent Office
Office européen des brevets

(11) Veröffentlichungsnummer: **0 415 219 A1**

(12) **EUROPÄISCHE PATENTANMELDUNG**

(21) Anmeldenummer: **90115896.4**

(22) Anmeldetag: **20.08.90**

(51) Int. Cl.$^5$: **C07K 7/52**, C12P 21/04, A61K 37/02, //(C12P21/04, C12R1:465)

(30) Priorität: **01.09.89 CH 3168/89**
**16.02.90 CH 503/90**

(43) Veröffentlichungstag der Anmeldung:
**06.03.91 Patentblatt 91/10**

(84) Benannte Vertragsstaaten:
**AT BE CH DE DK FR GB IT LI NL**

(71) Anmelder: **F. HOFFMANN-LA ROCHE AG**
**Postfach 3255**
**CH-4002 Basel(CH)**

(72) Erfinder: **Clozel, Jean-Paul, Dr.**
**11 Rue Oberlin**
**F-68300 St. Louis(FR)**
Erfinder: **Fischli, Walter, Dr.**
**Burgfeldermattweg 53**
**CH-4123 Allschwil(CH)**
Erfinder: **Hochuli, Erich, Dr.**
**Kirchackerstrasse 25**
**CH-4411 Arisdorf(CH)**
Erfinder: **Kupfer, Ernst, Dr.**
**Witikonerstrasse 70**
**CH-8032 Zurich(CH)**
Erfinder: **Weber, Wolfgang, Dr.**
**Röttler Ring 7**
**W-7889 Grenzach-Wyhlen(DE)**
Erfinder: **Weibel, Ernst Karl, Dr.**
**Vogtacherweg 3**
**CH-4133 Pratteln(CH)**

(74) Vertreter: **Mezger, Wolfgang, Dr. et al**
**Grenzacherstrasse 124 Postfach 3255**
**CH-4002 Basel(CH)**

(54) **ANF-Antagonist.**

(57) Die Erfindung betrifft aus Mikroorganismen der Gattung Streptomyces mit den DSM-Nrn. 4777 und 4778 isolierbare Antagonisten des Säuger-atrialen natriuretischen Faktors und physiologisch verträgliche Salze davon wie Verfahren zur Isolierung solcher Antagonisten und Herstellung deren Salze.

EP 0 415 219 A1

## ANF-ANTAGONIST

Gegenstand der vorliegenden Anmeldung ist ein Antagonist des Säuger-atrialen natriuretischen Faktors, dessen physiologisch verträgliche Salze. Mikroorganismen, die besagten Antagonisten enthalten, Verfahren zur Herstellung des Antagonisten und seiner Salze, pharmazeutische Zusammensetzungen. die einen solchen Antagonisten oder dessen Salze enthalten und deren Verwendung zur Behandlung von Krankheiten, insbesondere solchen, bei denen die Regulation des Blutdruckes eine Rolle spielt.

Der atriale natriuretische Faktor (ANF) selbst wird als Prä-Pro-Hormon im Säuger-Atrium synthetisiert und als Pro-Hormon von 126 Aminosäuren in Granulen gespeichert. Durch bestimmte Stimuli, wie z.B. Dehnung des Vorhofes, wird das Protein zum biologisch aktiven Peptid, bestehend aus 28 Aminosäuren (99-126), prozessiert und in den Blutkreislauf sezerniert.

Das bevorzugte Zielorgan des ANF ist die Niere, wo er die glomeruläre Filtrationsrate, den renalen Blutfluss, und die Natriumexkretion verstärkt und das Urinvolumen vergrössert. Darüberhinaus wirkt ANF auch blutgefässerweiternd, was zu einer Senkung des Blutdruckes führt. Generell scheint ANF die Regulationsmechanismen des Blutdruckes zu beeinflussen [Needleman, P. und Greenwald, J.E., New Engl. J. Med. 314, 828 (1986)].

Andererseits antagonisiert ANF die physiologischen Effekte des Renin-Angiotensin-Systems und zwar auf verschiedenen Ebenen: durch Reduktion der Renin-Sekretion, durch Relaxation der Blutgefässe, die durch Angiotensin vorkontrahiert sind, durch Blockade der Angiotensin-induzierten Aldosteron Synthese und durch die natriuretischen und diuretischen Effekte, die die Aldosteron-induzierte Natrium-Retention aufheben.

Es wurde nun ein ANF-Antagonist gefunden. der bei der akuten Bekämpfung von schweren Hypotensionen. beispielsweise in Schockzuständen, und zu Dehydratationen verwendet werden kann.

Zur Herstellung eines erfindungsgemässen ANF-Antagonisten kann dieser aus Mikroorganismen der Gattung Streptomyces, bevorzugt aus Streptomyceten. die am 30.8.1988 bei der Deutschen Sammlung für Mikroorganismen in Braunschweig mit den DSM-Nrn. 4777, 4778 nach dem Budapester Vertrag hinterlegt worden sind sowie aus deren ANF-Antagonisten enthaltenden Subkulturen, Mutanten und Varianten isoliert werden.

Dazu können besagte Mikroorganismen nach bekannten Fermentationsmethoden (z.B. Rehm, H.J. "Industrielle Mikrobiologie". Springer-Verlag. Berlin, Heidelberg. New York, 1980) in flüssigen Medien, beispielsweise solchen, die Stärke, Dextrin, Glucose, D-Mannitol, Ribose oder Glycerin als Kohlenstoffquelle und Sojamehl, Hefeextrakt oder Pepton als Stickstoffquelle enthalten, kultiviert werden. Als Salze für besagte Medien kommen vorzugsweise Ammonium-, Magnesium-oder Calciumsalze oder Gemische davon in Frage. Als weitere Fermentationsbedingungen sind ein Temperaturbereich von ungefähr 20-37° C, ein Inkubationszeitraum von 1-6 Tagen unter aeroben Bedingungen und die Wahl eines bekannten geeigneten Antischaummittels, wie beispielsweise eines solchen auf Polypropylenbasis, anzusehen.

Aus besagten, unter den angegebenen Bedingungen kultivierten Mikroorganismen kann ein ANF-Antagonist entweder aus dem Rohextrakt, d.h. einem Extrakt aus Zellen und Kulturmedium, aus der Zellmasse oder aus dem Kulturfiltrat durch Kombination von dem Fachmann bekannten Methoden der Peptidreinigung isoliert werden. Zum Nachweis eines solchen bei Säugern wie Mensch, Rind oder Ratte wirkenden ANF-Antagonisten kann eine Kombination der in den Beispielen 4 und 5 im Detail beschriebenen Methoden, bzw. Bestimmung einer verminderten Syntheserate von cyclischem Guanosinmonophosphat oder andere dem Fachmann geläufige Nachweismethoden für Antagonisten verwendet werden.

Als bevorzugte Methoden zur Reinigung eines ANF-Antagonisten aus besagtem Rohextrakt kommen die folgenden in Betracht: Ultrafiltration, flüssig-flüssig-Extraktion, z.B. mit einem Gemisch aus Wasser und 2-Butanol, Anionenaustauscher-Chromatographie, bevorzugt an DEAE-Sepharose; Kieselgelchromatographie (inkl. an Umkehrphasen). Affinitätschromatographie, beispielsweise an einem Chelat-Affinitätsharz, bevorzugt an einem Kupfer-Chelat-Harz, besonders bevorzugt an einem Harz mit einem Iminodiessigsäureliganden und Gelpermeationschromatographie an modifiziertem Dextran, beispielsweise Sephadex LH-20.

Aus der Zellmasse kann ein ANF-Antagonist mittels bekannter Methoden der Peptidreinigung, bevorzugt durch Extraktion mit niederkettigem Alkohol, beispielsweise Methanol, gereinigt werden.

In einer bevorzugten Ausführungsform wird ein ANF-Antagonist aus dem Kulturfiltrat isoliert. Dazu können ebenfalls bekannte Methoden der Protein- bzw. Peptidreinigung wie beispielsweise Adsorption an einem makroporösen Adsorberharz, vorzugsweise auf Polystyrolbasis, z.B. Amberlite, Ionenaustauscherchromatographie, vorzugsweise auf Agarosebasis, z.B. DEAE-Sepharose, Chromatographie an Kieselgel einschliesslich an Umkehrphasen und Affinitätschromatographie, vorzugsweise an einer Metall-Chelat-Matrix, sowie Extraktionsverfahren, wovon Extraktion mit 2-Butanol besonders bevorzugt ist, verwendet

werden.

In einer besonders bevorzugten Ausführungsform wird ein ANF-Antagonist in folgender Weise isoliert. Der ANF-Antagonist wird aus dem Kulturfiltrat mittels eines makroporösen Adsorberharzes auf Polystyrolbasis abgetrennt und von diesem Harz mittels wässrigen Isopropanols eluiert. Das biologisch aktive Eluat dieser Säule wird dann nach Zentrifugation, Verdünnung mit Phosphatpuffer und Filtrierung auf eine mit Phosphatpuffer, pH 7,0 äquilibrierte DEAE-Ionenaustauschersäule gegeben. Elution des ANF-Antagonisten erfolgt mit einer geeigneten Konzentration an NaCl. Das so erhaltene Eluat wird dann auf eine Umkehrphasenkieselgelsäule gepumpt. Elution des ANF-Antagonisten davon erfolgt mittels eines Stufengradienten aus Wasser/Acetonitril, der eine geeignete Menge an Trifluoressigsäure enthält. Dieses mit Wasser verdünnte Eluat wird dann auf eine präparative C-18 Kieselgelsäule gegeben. Elution des ANF-Antagonisten von dieser Säule erfolgt mittels eines linearen Wasser/Acetonitrilgradienten. Das Eluat wird in Anwesenheit von Isopropanol eingedampft. Der Rückstand wird in Isopropanol aufgenommen, auf einer mit Phosphatpuffer/NaCl äquilibrierten Kupfer-Chelatgelsäule aufgetrennt und der ANF-Antagonist mit Aequilibrierpuffer und einer geeigneten Konzentration an Imidazol eluiert. Dieses Eluat wird auf eine präparative C-18 HPLC-Kieselgelsäule gegeben. Mittels eines linearen Acetonitrilgradienten in Wasser bei Anwesenheit einer geeigneten Trifluoressigsäurekonzentration kann der ANF-Antagonist in reiner Form davon eluiert werden.

Die Charakterisierung eines erfindungsgemässen ANF-Antagonisten kann mittels der in der Peptidchemie üblichen physikalisch-chemischen Methoden durchgeführt werden. Die Figuren 1-3 zeigen spezifische Daten eines gemäss den Beispielen 1-5 isolierten ANF-Antagonisten.

Figur 1 : "Fast atomic bombardment"-Massenspektrum des ANF-Antagonisten in Thioglycerin; m/z = 1870,9 ist der $(M+H^+)$-Scheitelwert mit der Zusammensetzung $^{12}C_{90}H_{111}N_{21}O_{24}+H^+$. Das berechnete Molekulargewicht betrug 1870,8.

Figur 2 : Infrarotspektrum des ANF-Antagonisten in KBr.

Figur 3 : Ultraviolettspektrum [in Methanol gemessen; $\lambda_{max}$ = 280 nm ($\epsilon$ = 6923)].

Des weiteren wurde für diesen ANF-Antagonisten ein optischer Drehwert von $[\alpha]_D^{25} = +12^\circ$ (c=0,9%, in Methanol) bestimmt. Ab etwa 200°C erfolgte Zersetzung dieses ANF-Antagonisten. Charakterisierung mittels Dünnschichtchromatographie, analytischer Hochdruckflüssigchromatographie, Aminosäureanalyse und NMR-Spektroskopie kann in bekannter Weise und bevorzugt wie im Detail in Beispiel 3 beschrieben, durchgeführt werden. Die spezifischen Daten dieser Analysen befinden sich ebenfalls in Beispiel 3.

Auf Grund der ermittelten Daten handelt es sich bei dem erfindungsgemässen ANF-Antagonisten um ein cyclisches Peptid.

Cyclische Peptide können mittels in der Peptidchemie bekannter Methoden, wie beispielsweise durch klassische Methoden der Peptidsynthese in Lösung, durch Kondensation von Fragmenten, durch teilweise oder vollständige Festphasen-Synthese, beispielsweise wie von Merrifield in J. Am. Chem. Soc. 85 , 2149 (1963) beschrieben, und anschliessende Cyclisierung chemisch synthetisiert werden. Im übrigen können auch die linearen Analogen solcher cyclischen Peptide durch Anwendung der dem Fachmann bekannten Methoden der rekombinanten DNA-Technologie, wie beispielsweise in Maniatis et al. (1982) "Molecular Cloning". Cold Spring Harbor beschrieben, hergestellt werden und anschliessend cyclisiert werden.

Ein weiterer Gegenstand der vorliegenden Erfindung sind Analoge des aus Streptomyceten erhaltenen ANF-Antagonisten mit ANF antagonisierender Wirkung. Unter Analogen sind solche Verbindungen zu verstehen, bei denen entweder eine oder mehrere Aminosäuren des cyclischen Peptids in bekannter Weise an den Seitengruppen chemisch modifiziert sind oder solche, bei denen eine oder mehrere Aminosäuren ausgetauscht sind oder fehlen, ohne dass dadurch die ANF antagonisierende Wirkung beeinträchtigt wird. Solche Analoge können nach den bereits beschriebenen Methoden der Peptidchemie oder der rekombinanten DNA-Technlogie, wie beispielsweise gezielte Mutagenese, hergestellt werden.

Der erfindungsgemässe ANF-Antagonist und seine Analogen sowie deren physiologisch verträgliche Salze können zur Herstellung von pharmazeutischen Präparaten. vor allem solchen zur Behandlung von Krankheiten, bei denen die Regulation des Blutdruckes eine Rolle spielt, verwendet werden. Dazu kann ein erfindungsgemässer ANF-Antagonist - falls wünschenswert bzw. erforderlich in Verbindung mit anderen pharmazeutisch aktiven Substanzen - mit den üblicherweise verwendeten festen oder flüssigen Trägermaterialien in bekannter Weise verarbeitet werden. Die Dosierung solcher Präparate kann unter Berücksichtigung der üblichen Kriterien in Analogie zu bereits verwendeten Präparaten, ähnlicher Aktivität und Struktur erfolgen.

Nachdem die Erfindung im Vorhergehenden im allgemeinen beschrieben worden ist, sollen die folgenden Beispiele die Erfindung mehr im Detail veranschaulichen, wobei sie in keiner Weise den Gegenstand der vorliegenden Erfindung einschränken sollen.

Beispiel 1

Fermentation

10 Schüttelkolben mit jeweils 100 ml Medium 644 (2% Sojamehl vollfett, 2% D-Mannitol; pH wurde vor der 20-minütigen Sterilisation des Mediums bei 121 °C mit NaOH auf 7,4 eingestellt; ebenfalls vor der Sterilisation wurden dem Medium Polypropylenglycol in einer Konzentration von 1 ml/l zugegeben) wurden mit Sporen oder Myzel von dem Streptomyceten mit der DSM-Nr. 4778) beimpft und 48 Stunden aerob bei 30 °C inkubiert. Diese Kulturen wurden dann in einen Fermenter überimpft, der 10 l Medium 644 enthielt und während 24 Stunden bei 30 °C, einer Belüftung von 0,4 vvm und Rühren mit 360 rpm gefahren wurde. Dieser Fermenter wurde dann zum Beimpfen eines 200 l Medium 644 enthaltenden Produktionsfermenters verwendet. Dieser wurde 96 Stunden bei 30 °C, einer Belüftung von 0.3 vvm und Rühren mit 600 rpm gefahren.

5 Fermentationen zu je 200 l, die auf diese Weise durchgeführt wurden, ergaben zusammen eine Ausbeute von 6,1 g ANF-Antagonist (bestimmt mittels des Bindungstests aus Beispiel 4) im Kulturfiltrat.

Beispiel 2

Isolierung des ANF-Antagonisten

Stufe 1:

Die Gärbrühe eines 200 l Fermenters gemäss Beispiel 1 wurde über eine Nutsche filtriert, was 130 l Kulturfiltrat ergab.

Stufe 2:

Das Filtrat von Stufe 1 wurde mit 20 l/h durch eine mit Servachrom XAD-2 (Serva, Heidelberg, Deutschland, Korngrösse 0,3-0,9 mm) gefüllte Chromatgraphiesäule gepumpt (Bettdimension: 15 x 50 cm) und anschliessend mit 20 l Wasser und 20 l 10%igem wässig. Isopropanol gewaschen. Der ANF-Antagonist wurde mit 25 l 50% Isopropanol vom Harz eluiert. Das Eluat wurde bei 20 °C im Vakuum eingeengt und ergab 1 l biologisch aktives Konzentrat. Das Adsorberharz wurde durch aufeinanderfolgendes Waschen mit 10 l Isopropanol, 10 l 50% Isopropanol und 30 l Wasser regeneriert.

Stufe 3:

Zur weiteren Reinigung wurde eine Chromatographiesäule mit 4 l DEAE-Sepharose FF (Pharmacia, Uppsala, Schweden) gefüllt (Bettdimension: 14 x 26 cm) und mit 12 l 100 mM Natriumphosphat-Puffer (pH 7,0). gefolgt von 8 l 10 mM Natriumphosphat-Puffer (pH 7,0) gewaschen. Die Säule wurde mit einem Fluss von 4-5 l/Std. betrieben. 1 l des Konzentrats von Stufe 2 wurde mit 7 l 10 mM Natriumphosphat-Puffer (pH 7,0) verdünnt, zentrifugiert (30 Min., 10'000 rpm), filtriert (Faltenfilter) und durch die Säule gepumpt. Die Säule wurde dann mit 16 l 10 mM Natriumphosphat-Puffer (pH 7,0) gewaschen, und der ANF-Antagonist mit 16 l 10 mM Natriumphosphat-Puffer (pH 7,0), 100 mM Natriumchlorid eluiert. Zur Regeneration des Ionenaustauschers wurde die Säule mit 8 l 10 mM Natriumphosphat-Puffer (pH 7,0), 1 M Natriumchlorid, gefolgt von 8 l 0.1 M Natronlauge, gewaschen.

Fünf Reinigungsläufe über die Stufen 1 bis 3 ergaben 62 l ANF-Antagonist enthaltendes Eluat.

Stufe 4:

62 l Eluat der Stufe 3 wurden durch eine Chromatographiesäule gepumpt, die mit Silica RP-18 von ICN Biomedicals (Eschwege, Deutschland) gefüllt worden war (Korngrösse: 32-63 µm; Bettdimensionen: 3,7 x 58 cm; Fluss: 3.6 l/Std.). Anschliessend wurde die Säule der Reihe nach mit 1 l Wasser, 8.5 l 0,1 M Natriumphosphat-Puffer (pH 3,0) und 4 l Wasser gewaschen. Die Säule wurde dann mit einem Stufengradienten von Wasser/Acetonitril, 5 mM Trifluoressigsäure gewaschen, wobei der ANF-Antagonist bei 40-60% Acetonitril eluiert wurde. Man sammelte 5.7 l Eluat.

Stufe 5:

Das Eluat von Stufe 4 wurde mit Wasser auf 10 l verdünnt und durch eine Stahlsäule, die mit Prep C-18 (Waters, Milford, USA) gefüllt worden war, gepumpt (Korngrösse: 50-105 µm; Bettdimension: 5 x 30 cm; Fluss: 80 ml/min.). Dann wurde ein Gemisch von Wasser/Acetonitril, das 5 mM an Trifluoressigsäure war, durch die Säule gepumpt. Während 25 Minuten betrug der Acetonitrilanteil 20%. während 30 Minuten 20-40% (linearer Gradient) und schliesslich während 75 Minuten 40%, wobei der ANF-Antagonist eluiert wurde. Man sammelte 1,21 l Eluat, das bei 40°C im Vakuum eingedampft wurde, wobei portionenweise Isopropanol zugegeben wurde, um das Produkt in Lösung zu halten. Es wurden 3,99 g Eindampfrückstand erhalten, die 2.44 g ANF-Antagonist (bestimmt durch Aminosäureanalyse) entsprachen.

Stufe 6:

Aus Sepharose CL 6B FF wurde nach Hochuli, E. [Chimica 40, 408 (1986)] ein Chelatgel der folgenden Struktur hergestellt:

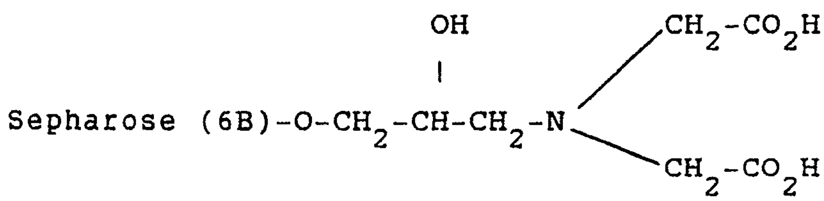

0.8 l davon wurden in eine Chromatographiesäule gefüllt (Bettdimensionen: 7 x 21 cm). Zur Vorbereitung wurde das Gel der Reihe nach bei einem Fluss von 5 l/h mit folgenden Lösungen gewaschen: 2 l 50 mM Kupfersulfatlösung: 2 l Wasser; 2 l 50 mM Natriumacetatlösung (pH 3,5). 1 M Natriumchlorid; 2 l Wasser und 2 l 50 mM Natriumphosphat-Puffer (pH 7,5), 0.5 M Natriumchlorid (Aequilibrier-Puffer). 2 g des in Stufe 5 erhaltenen Materials wurden in 75 ml Isopropanol gelöst, mit Aequilibrier-Puffer auf 1 l verdünnt und bei einem Fluss von 2 l/h durch die Chelatgelsäule gepumpt. Anschliessend wurde mit 4 l Aequilibrierpuffer gewaschen. Der ANF-Antagonist wurde dann mit 4,4 l Aequilibrierpuffer, der 3 mM an Imidazol war, gefolgt von 2,6 l Aequilibrierpuffer, der 6 mM an Imidazol war, bei einem Fluss von 2 l/h eluiert. Aus den gesamten 3.99 g des Materials von Stufe 5 erhielt man in 2 Läufen auf der Chelatsäule 3,4 l Eluat, das gemäss HPLC-Analyse (s. Beispiel 3) nur ANF-Antagonist enthielt. Zur Regeneration wurde das Chelatgel mit folgenden Lösungen bei einem Fluss von 5 l/Std. gewaschen: 2 l 50 mM EDTA-Lösung (pH 8.0), 2 l Wasser; 2 l 0.2 M Natronlauge; 5 l Wasser.

Stufe 7:

Das Eluat der Stufe 6 (3,4 l) wurde durch eine mit Prep C-18 (Waters) gefüllte Stahlsäule gepumpt (gleiche Versuchsparameter wie bei Stufe 5). Anschliessend wurde die Säule mit mM Trifluoressigsäure gewaschen, bis das Eluat einen sauren pH-Wert anzeigte. Der ANF-Antagonist wurde dann mit einem linearen Gradienten von 0-80% Acetonitril/5 mM Trifluoressigsäure in 60 Minuten von der Säule eluiert. Man sammelte 250 ml Eluat, das bei 40°C im Vakuum eingeengt wurde und nach dem Lyophilisieren 1,25 g ANF-Antagonist als weisses Pulver ergab.

Beispiel 3

Charakterisierung des ANF-Antagonisten

Dünnschichtchromatographie: Auf Kieselgel 60 $F_{254}$ (Merck) wurde mit n-Butanol/Essigsäure/Wasser als Laufmittel im Verhältnis 80/20/20 (v/v/v) ein Fleck bei $R_f$ = 0.66, der mit Chlor/Tolidin anfärbte, erhalten.

Hochdruckflüssigkeitschromatographie (HPLC): Auf einer µBondapak C-18-Säule (3,9 x 300 mm) von Waters wurde mit einem Laufmittelgemisch aus Acetonitril/Wasser im Verhältnis 1:1, 5 mM Trifluoressigsäure bei einem Fluss von 1 ml/min. und Nachweis bei 228 nm ein Signal mit einer Retentionszeit von 4,46 min. erhalten.

Die Aminosäureanalyse wurde nach Spackman, D.H. et al. [Analyt Chem. 30 , 1190 (1958)] durchgeführt. Die Hydrolysen wurden mit 6 N HCl bei 110°C in 24 Stunden oder mit 4 N Methansulfonsäure durchgeführt. Die Bestimmung der Aminosäurezusammensetzung des Hydrolysats ergab mit dem Liquimat III Aminosäureanalysator (Kontron AG) folgendes Resultat:

| Aminosäure | gefundene Menge | Theoretische Menge |
|---|---|---|
| His | 0,96 | 1 |
| $NH_3$ | 1,23 | |
| Asx | 3,00 | 3 |
| Ser | 0,85 | 1 |
| Gly | 4,99 | 5 |
| Ile | 1,92 | 2 |
| Tyr | 1,02 | 1 |
| Phe | 2,96 | 3 |
| Trp | 1,1 | 1 |

Der Wert für Asx wurde gleich 3 gesetzt. Spezielle Analysen, die ebenfalls nach Spackman, D.H. et al. [Analyt. Chem. 30 , 1190 (1958)] durchgeführt wurden, ergab für Asx = 2 Asp und 1 Asn. Die Konfiguration der Aminosäuren wurde durch Hydrolyse und anschliessende Derivatisierung mit (+)-1-(9-Fluorenyl)-ethylchlorformiat als (L)-Konfiguration bestimmt.

Die Primärstruktur wurde durch automatisierten Edman-Abbau von durch enzymatische wie chemische Spaltung erhaltenen Peptiden, durch NMR-Spektroskopie des Gesamtmoleküls unter gemeinsamer Verwendung verschiedener 2D-Pulstechniken wie "ROESY, "NOESY" und "RELAYED COSY" und durch "FAB"-Massenspektroskopie ermittelt. Die Struktur ist unter Verwendung der in der Peptidchemie üblichen Abkürzungen für Aminosäuren in Figur 4 dargestellt. Es handelt sich dabei um ein substituiertes 1,4,7,10,13,16,19,22-Octaazacyclopentacosan.

## Beispiel 4

In vitro Nachweis des ANF-Antagonisten

In einem Bindungstest nach Bürgisser et al. [Biochem. Biophys. Res. Comm. 133 , 1201 (1985)] wurde die Verdrängung von radioaktiv-markiertem ANF von dessen Rezeptor durch den ANF-Antagonisten gemessen. Als Rezeptorpräparation wurde eine Membranpräparation von Rindernebennieren verwendet. Zu 100 µl dieser Präparation in 50 mM Tris/HCl, 500 mM $MgCl_2$, 1 mM EDTA. 0,5% Rinderserumalbumin und 1 mM o-Phenanthrolin, pH 7,6, wurden $^{125}$I-ANF (20'000 cpm. 18,2 pM) in 145 µl dieses Puffers und der Ligand in 5 µl Dimethylsulfoxid gegeben. Diese Mischung wurde 24 Stunden bei 4°C inkubiert und gebundenes von freiem ANF durch schnelle Filtration über Whatman GF/C Glasfaserfilter abgetrennt. Nach dreimaligem Waschen der Filter mit obigem Puffer (3 x 4 ml) wurden die Filter zerschnitten und deren Radioaktivität im Gamma-Zähler gemessen. Die Inhibition der Bindung wurde quantitativ mit der $IC_{50}$ erfasst, worunter diejenige Konzentration an entsprechendem Liganden zu verstehen ist, bei der die

Bindung von $^{125}$I-ANF halbmaximal gehemmt wird.

Tabelle

| IC$_{50}$-Werte | |
|---|---|
| Ligand | IC$_{50}$ (nM) |
| Ratten ANF (99-126) | 0,110 |
| ANF-Antagonist | 860 |

## Beispiel 5

### In vivo Wirkung des ANF-Antagonisten

Für die in vivo Versuche wurden 20 Wochen alte spontanhypertensive Ratten (SHR) oder normotensive pathogen-freie (SPF) Ratten verwendet. In wache SHR wurde 2.5 µg/kg/min ANF infundiert, was den Blutdruck um 45 mm Hg senkte. Eine anschliessende Bolusgabe von 1 mg/kg ANF-Antagonist erhöhte den Blutduck wiederum auf den Ausgangswert von 220 mm Hg. Anästhesierten SPF-Ratten ohne zentrale Reflexe ("pithed rats") wurde Angiotensin II (0,15 µg/kg/min) infundiert, um den Blutdruck auf 100-110 mm Hg anzuheben. Dann wurde ein Bolus von 10 µg/kg ANF gegeben. was den Blutdruck um 20 mm Hg senkte. Eine anschliessende intravenöse Gabe von 1 mg/kg ANF-Antagonist erhöhte den Blutdruck wiederum auf den Ausgangswert. Diese Versuche zeigen, dass der Blutdruckanstieg unter dem ANF-Antagonisten nicht auf eine Aktivierung von symphatischen Reflexen, sondern auf die Wirkung eines ANF-Antagonisten zurückzuführen ist.

## Beispiel 6

### Dimethylester des ANF-Antagonisten

40 mg des gemäss Beispiel 2 isolierten ANF-Antagonisten wurden in 40 ml Methanol, der 30 mg 96%ige Schwefelsäure enthielt, gelöst und 65 Stunden bei Raumtemperatur stehen gelassen. Danach wurde die Lösung mit 120 ml Wasser verdünnt und bei einem Fluss von 5 ml/min auf eine mit Nucleosil C-18, 10 µm (von Macherey-Nagel, Düren, Deutschland) gefüllte Stahlsäule (2 x 25 cm) gepumpt. Anschliessend wurde die Säule mit 30% Acetonitril in Wasser, 5 mM Trifluoressigsäure gewaschen. Der Dimethylester wurde mit 50% Acetonitril in Wasser, 5 mM Trifluoressigsäure eluiert. Das Eluat wurde lyophilisiert und ergab 31 mg des Dimethylesters als farbloses Pulver. Der gemäss Beispiel 4 bestimmte IC$_{50}$-Wert des Dimethylesters betrug 2500 nM.

## Ansprüche

1. Ein Antagonist des Säuger-atrialen natriuretischen Faktors, isolierbar aus Mikroorganismen der Gattung Streptomyces mit den DSM-Nrn. 4777 und 4778.
2. Eine Verbindung gemäss Anspruch 1, dadurch gekennzeichnet, dass diese ein cyclisches Peptid mit einem Molekulargewicht von etwa 1870 ist oder ein physiologisch verträgliches Salz eines solchen Peptids.
3. Eine Verbindung gemäss Anspruch 2 mit der in Figur 4 dargestellten Aminosäuresequenz.
4. Eine Verbindung gemäss einem der Ansprüche 1-3 als therapeutisch wirksames Mittel.
5. Eine Verbindung gemäss einem der Ansprüche 1-3 zur Behandlung von Krankheiten, bei denen die Regulation des Blutdruckes eine Rolle spielt.

6. Ein Verfahren zur Herstellung einer Verbindung gemäss einem der Ansprüche 1-3, dadurch gekennzeichnet, dass man Mikroorganismen der Gattung Streptomyces der DSM-Nrn. 4777 und 4778 kultiviert und dann eine Verbindung gemäss wenigstens einem der Ansprüche 1-3 aus einem Rohextrakt der Kultur, der Kulturzellmasse oder dem Kulturfiltrat isoliert und gewünschtenfalls in ein physiologisch verträgliches Salz überführt.

7. Pharmazeutische Präparate, dadurch gekennzeichnet, dass sie eine Verbindung gemäss einem der Ansprüche 1-3, gewünschtenfalls in Kombination mit einer oder mehreren therapeutisch wirksamen Substanzen und/oder nicht-toxischen, inerten, therapeutisch verträglichen Trägermaterialien enthalten.

8. Pharmazeutische Präparate zur Behandlung von Krankheiten, bei denen die Regulation des Blutdruckes eine Rolle spielt, wobei solche Präparate dadurch gekennzeichnet sind, dass sie eine Verbindung gemäss einem der Ansprüche 1-3, gewunschtenfalls in Kombination mit einer oder mehreren therapeutisch wirksamen Substanzen und/oder nicht-toxischen, inerten, therapeutisch verträglichen Trägermaterialien enthalten.

9. Verwendung einer Verbindung gemäss einem der Ansprüche 1-3 zur Behandlung von Krankheiten.

10. Verwendung einer Verbindung gemäss einem der Ansprüche 1-3 zur Behandlung von Krankheiten, bei denen die Regulation des Blutdruckes eine Rolle spielt.

11. Eine Verbindung gemäss einem der Ansprüche 1-3 wenn immer sie nach einem wie in Anspruch 6 beschriebenen Verfahren hergestellt wurde.

12. Die Erfindung wie zuvor beschrieben.

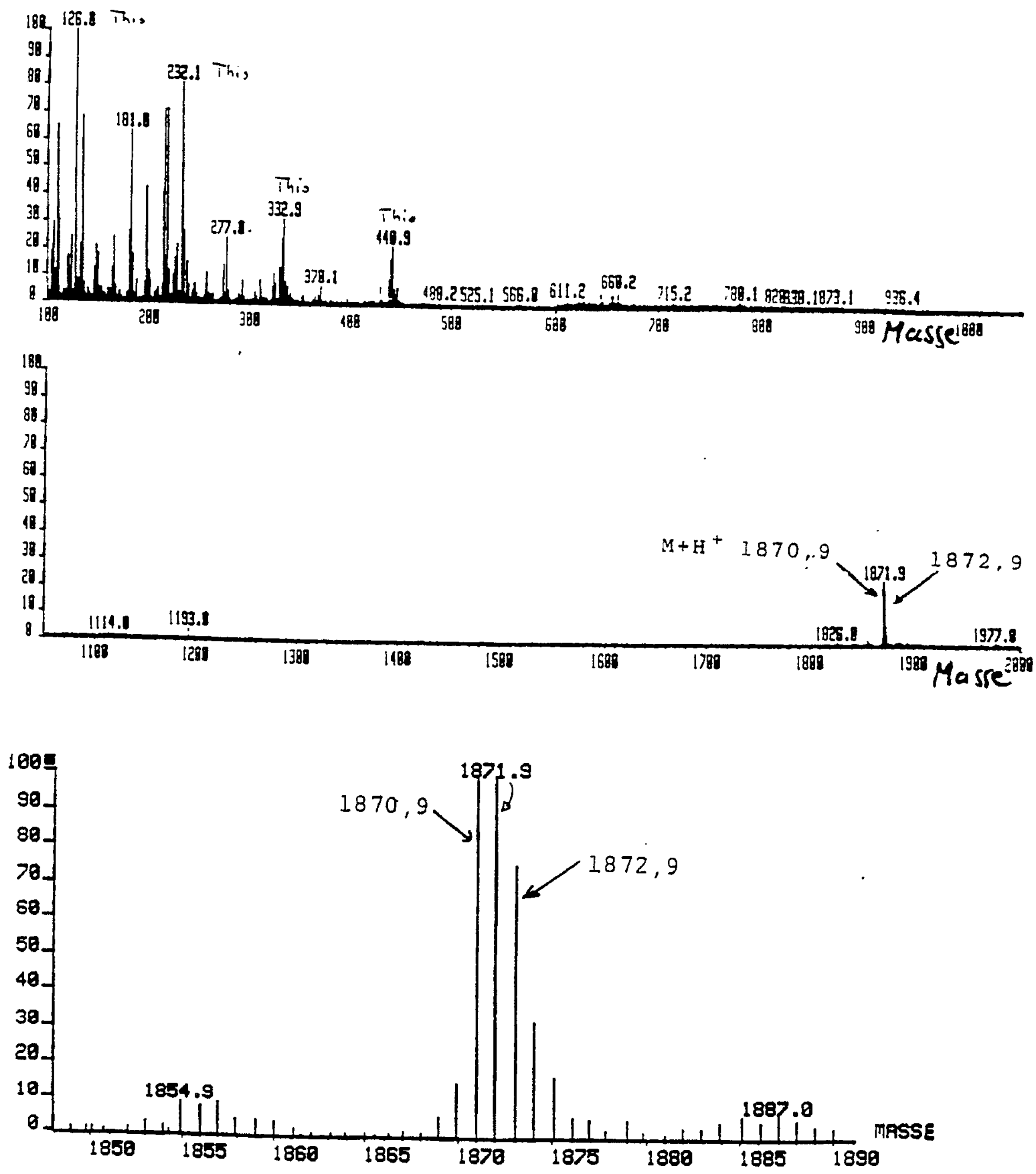
126.0 This
232.1 This
181.0
This
332.9
This
440.9
277.0
378.1
Masse
M+H+ 1870,9
1871.9
1872,9
1114.0
1193.0
1826.0
1977.0
Masse
1870,9
1871.9
1872,9
1854.9
1887.0
MASSE

Figur 1

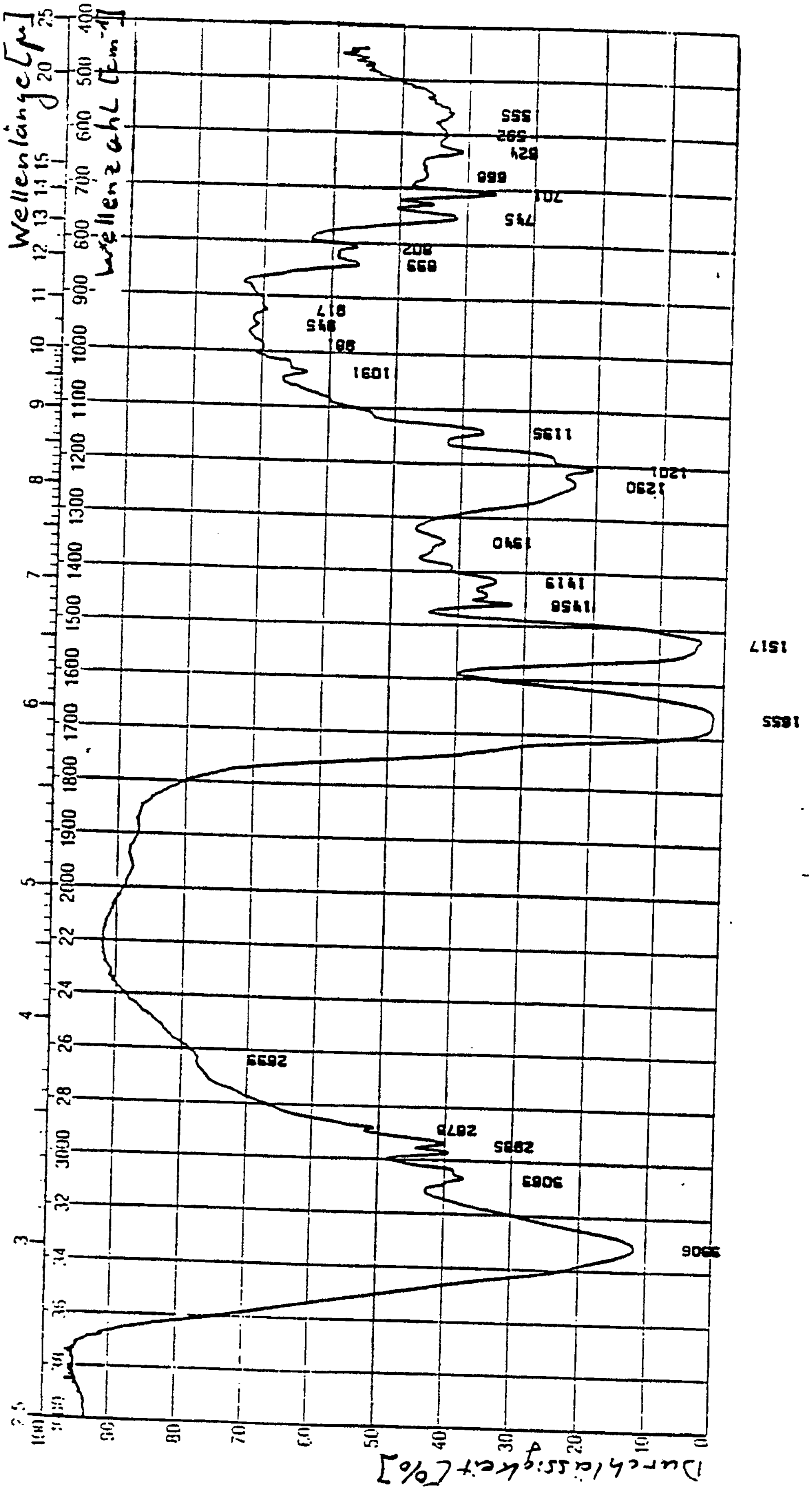
Wellenlänge [μ]
Wellenzahl [cm-1]
Durchlässigkeit [%]
555
592
624
668
701
745
802
899
817
845
881
1031
1195
1201
1290
1390
1419
1456
1517
1655
2699
2878
2985
3083
3306

Figur 2

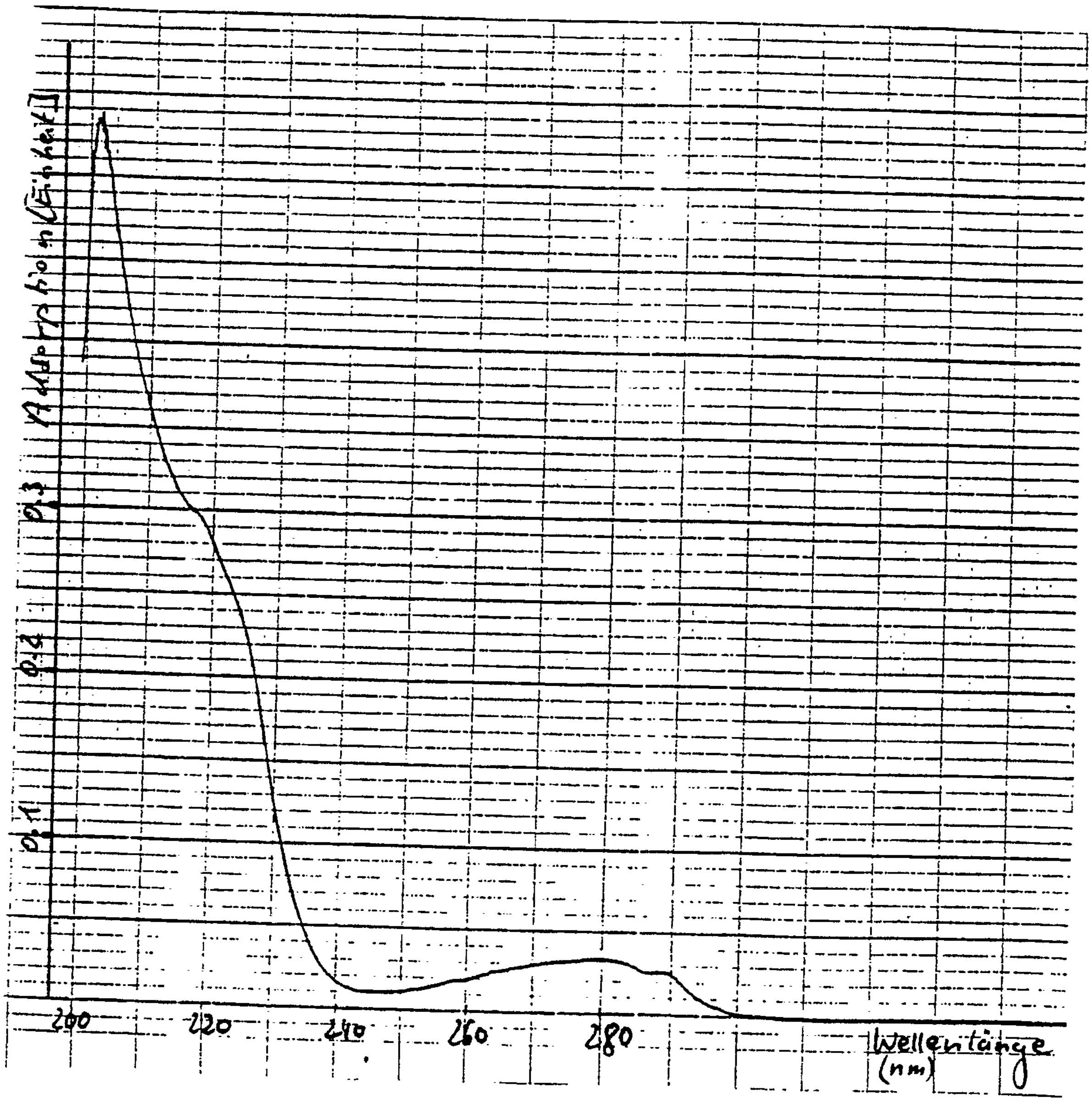
Adsorption [Einheit]
0.3
0.2
0.1
200
220
240
260
280
Wellenlänge
(nm)

Figur 3

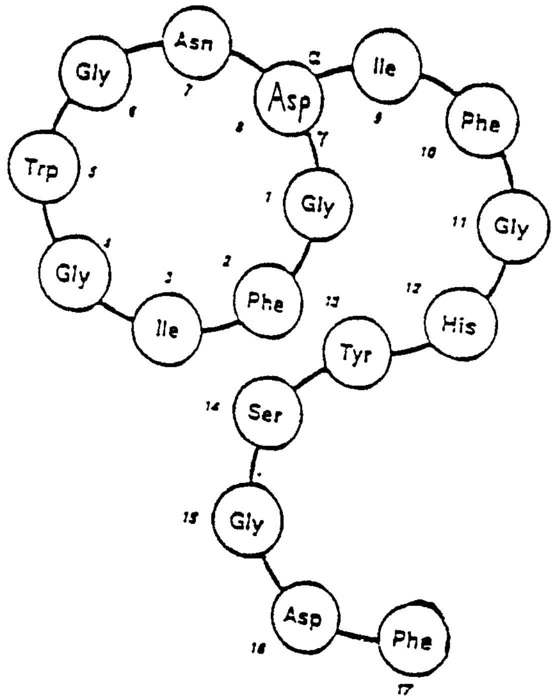
Gly
Asn
Asp
Ile
Phe
Trp
Gly
Gly
Gly
Ile
Phe
His
Tyr
Ser
Gly
Asp
Phe

Figur 4

Europäisches Patentamt

# EUROPÄISCHER RECHERCHENBERICHT

Nummer der Anmeldung

**EP 90 11 5896**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | BIOCHEMICAL AND BIOPHYSICAL RESEARCH COMMUNICATIONS, Band 164, 16. Oktober 1989, Seiten 108-113; T.J. ABELL et al.: "Competitive peptide antagonists of ANF-induced cyclic guanosine monophosphate production"<br>– – – – – | | C 07 K 7/52<br>C 12 P 21/04<br>A 61 K 37/02 //<br>(C 12 P 21/04<br>C 12 R 1:465) |

| RECHERCHIERTE SACHGEBIETE (Int. Cl.5) |
|---|
| C 07 K<br>C 12 P<br>A 61 K |

Der vorliegende Recherchenbericht wurde für alle Patentansprüche erstellt

| Recherchenort | Abschlußdatum der Recherche | Prüfer |
|---|---|---|
| Den Haag | 21 November 90 | NOVOA Y SANJURJO M.A |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument